# EUROPEAN PATENT SPECIFICATION

(11) **EP 3 640 642 B1**
(45) Date of publication and mention of the grant of the patent: **28.04.2021**
(21) Application number: 19204859.3
(22) Date of filing: 12.11.2015
(51) Int. Cl.: G01N 33/564, G01N 33/86, G01N 33/92

(54) **NOVEL ARTIFICIAL PHOSPHOLIPID-PROTEIN BIOCONJUGATES FOR BIOMOLECULAR RECOGNITION**
NEUARTIGE KÜNSTLICHE PHOSPHOLIPID-PROTEIN BIOKONJUGATE FÜR BIOMOLEKULARE ERKENNUNG
NOUVEAUX BIOCONJUGUÉS ARTIFICIELS DE LA PROTÉINE DE PHOSPHOLIPIDE POUR LA RECONNAISSANCE BIOMOLÉCULAIRE

(30) Priority: 26.11.2014 DK PA201470739; 11.05.2015 DK PA201570273
(43) Date of publication of application: 22.04.2020
(62) Divisional of application: 15797249.8
(73) Proprietor: Integrated biomedical technologies ApS, 2450 Copenhagen SV (DK)
(72) Inventor: ASTAKHOVA IRINA, Kira, 2450 Copenhagen SV (DK)
(74) Representative: Plougmann Vingtoft a/s

(56) References cited:
- WO-A1-2012/134925
- US-A1- 2006 234 392
- HANS-JÜRGEN MUSIOL ET AL: "Toward Semisynthetic Lipoproteins by Convergent Strategies Based on Click and Ligation Chemistry", CHEMBIOCHEM - A EUROPEAN JOURNAL OF CHEMICAL BIOLOGY., vol. 6, no. 4, 18 February 2005 (2005-02-18), pages 625-628, XP055242696, DE ISSN: 1439-4227, DOI: 10.1002/cbic.200400351
- GUBBENS J ET AL: "Photocrosslinking and Click Chemistry Enable the Specific Detection of Proteins Interacting with Phospholipids at the Membrane Interface", CHEMISTRY AND BIOLOGY, CURRENT BIOLOGY, LONDON, GB, vol. 16, no. 1, 30 January 2009 (2009-01-30), pages 3-14, XP025897056, ISSN: 1074-5521, DOI: 10.1016/J.CHEMBIOL.2008.11.009 [retrieved on 2009-01-30]
- FREDA H. PASSAM ET AL: "Laboratory tests for the antiphospholipid syndrome: current concepts", PATHOLOGY., vol. 36, no. 2, 1 April 2004 (2004-04-01), pages 129-138, XP055676499, AU ISSN: 0031-3025, DOI: 10.1080/00313020410001671966

## Description

### FIELD OF THE INVENTION

The present invention relates to phospholipid-peptide and phospholipid-protein conjugates useful for identifying autoantibodies.

### BACKGROUND OF THE INVENTION

Synthetic analogues of biomolecules encompass highly important and diverse tools for molecular recognition in research and clinical diagnostics of human diseases. Recently, significant progress has been made in the preparation of artificial nucleic acid and protein bioconjugates in context of synthetic probes for nanobiotechnology and biomolecular recognition. Furthermore, a novel copper-catalyzed and strain-promoted copper-free azide-alkyne cycloaddition (CuAAC and SPAAC) click chemistry reaction was proved to be efficient for bioconjugation giving stable products in excellent yields.⁴ However, CuAAC/SPAAC click chemistry has not been investigated in the field of bioconjugation of lipids.

Lipids are biomolecules which are involved in the most important processes in living organisms and therefore are of high importance for molecular diagnostics and studies of human diseases. Lipids are quite challenging objects for bioconjugation, since they are less soluble in aqueous media than other biomolecules and are more sensitive to oxidation.

Recent progress in bioconjugation strategies exemplified by CuAAC and SPAAC click chemistry reaction (M. G. Paulick, A. R. Wise, M. B. Forstner, J. T. Groves, C. R. Bertozzi. J. Am. Chem. Soc. 2007, 129, 11543) may form the basis for further advances in preparation of artificial lipid bioconjugates.

Although diagnostics of autoimmune disorders has been performed for the last 50 years, there are still many questions and challenges to be addressed. Thus, antibodies against ones own phospholipids are one of the biggest mysteries of human Antiphospholipide syndrome (APS), an autoimmune disorder with multiple manifestations and serious damage to the patients including thrombosis and recurrent pregnancy loss. Previously, diagnostics of APS required detection of antibodies against cardiolipin, a phospholipid which is an important component of the inner mitochondrial membrane in mammalians.

However, antibodies were found to be produced in patients with several viral as well as bacterial infections. Furthermore, recently it was demonstrated that some antibodies against cardiolipin are not directed to cardiolipin itself, but to the plasma protein beta2-GPI which binds to cardiolipin. Thus, modern diagnostics of APS involves separate tests on anti-cardiolipin, anti-beta2-GPI and antibodies against other phospholipids (including phosphatidylserine), and mixed phospholipids called lupus anticoagulant.

Such diagnostic approaches are time-consuming and expensive. Furthermore, current tests on anti-cardiolipin antibodies apply heterogeneous phospholipid molecules obtained from natural sources, which leads to low chemical stability, low selectivity and significant disagreement between results of different working groups.

WO 2012/134925 A1 describes compounds having a click-reactive group, a linker, and a carrier molecule comprising a nucleophilic group. The reactive group has greater hydrolytic stability than standard N-hydroxysuccinimidyl (SE) and perfluorophenolic (PFP) esters, that when attached via an intervening linker to a click-reactive group forms a click-labeling reagent US2006/234392 relates to an anti-phospholipid-cofactor protein-antibody detection reagent that comprises a phospholipid moiety, a cofactor protein, and a carrier The phospholipid can be phosphatidylethanolamine. Beta2-glycoprotein I or prothrombin can be conjugated via a succinimide carbodiimide reaction.

New synthetic analogues of cardiolipin and other phospholipids with high stability, sensitivity and selectivity of target antibodies' detection are highly desired in both research and clinical diagnostics of autoimmune disorders.

### SUMMARY OF THE INVENTION

The present invention provides novel phospholipid-peptide and phospholipid-protein conjugates with improved stability and sensitivity as well as selectivity of target antibodies' detection. These novel phospholipid-peptide and phospholipid-protein conjugates are useful for research and diagnostics of human autoimmune disorders. The invention is defined in its broadest sense in the appended claims.

In a first aspect of the disclosure there is provided a phospholipid protein conjugate of the formula: 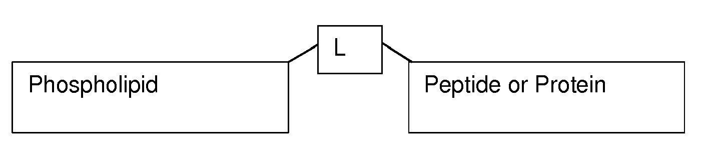 Phospholipid Peptide or Protein wherein L is a linker, such as 1,2,3-triazole, amide or their combination, and wherein the phospholipid and protein are biologically complementary, i.e. interact in vivo.

In an alternative aspect of the disclosure is provided a phospholipid protein conjugate of the formula (I): 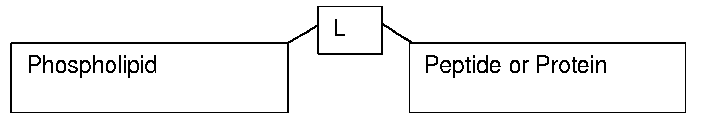 Phospholipid Peptide or Protein wherein L is a linker, such as 1,2,3-triazole, amide with a general formula RC(O)NHR' wherein R is a phospholipid and R' is protein/peptide; polyethyleneglycol (PEG) or their combination, and wherein the phospholipid and protein or a peptide are biologically complementary.

In the present invention the protein or shorter peptide moiety, such as between 10 to 100 amino acids, of the phospholipid-peptide and phospholipid-protein conjugates is selected from beta2-GPI and prothrombine. In the invention the present inventor has covalently bound via the 1,2,3-triazole linker biologically complementary molecules, namely phosphoethanolamine with human β-2-glycoprotein I and prothrombin. As described herein, the phospholipid is selected from cardiolipin. According to the invention, the phospholipid is a fatty acid derivative of phosphoethanolamin, i.e. 1 ,2-distearoyl-phosphoethanolamine. Proteins and peptide may be bound to the linker via the terminal amino terminus or amine containing amino acids such as e.g. lysine.

The resulting phospholipid-peptide and phospholipid-protein conjugates exhibit surprisingly high binding affinity and specificity for the autoimmune antibodies against autoimmune complexes. Accordingly, the conjugates are very useful in diagnostics and therapy of autoimmune diseases that involve the production of autoantibodies against the above mentioned phospholipids and proteins, such as antiphospholipid syndrome and systemic lupus erythematosus. The novel conjugates also surprisingly show improved stability upon storage.

### BIOCONJUGATES OF THE INVENTION

Conjugates of the present invention may be selected from the group consisting of formulas (BC-1) and (BC-2) below : 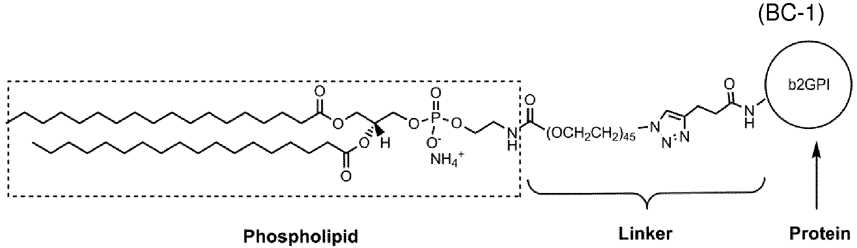 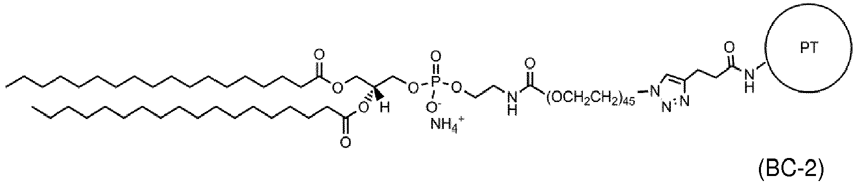 wherein b2GPI denotes b2-glycoprotein I and PT denotes prothrombin.

Further useful conjugates (not part of the invention) are compounds selected from the group consisting of: 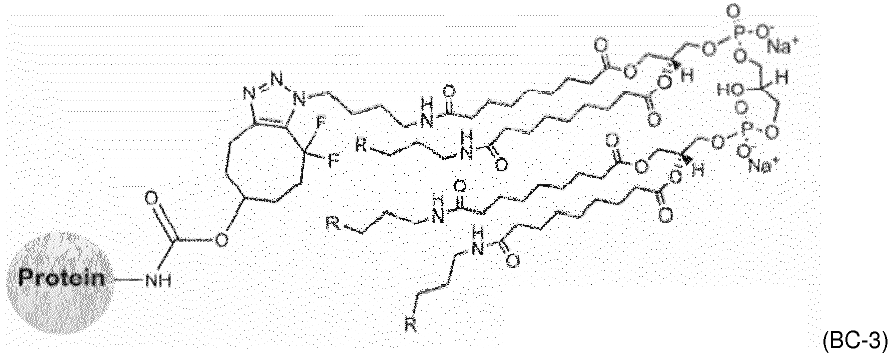 wherein: 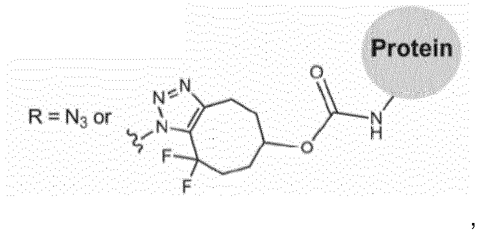 and 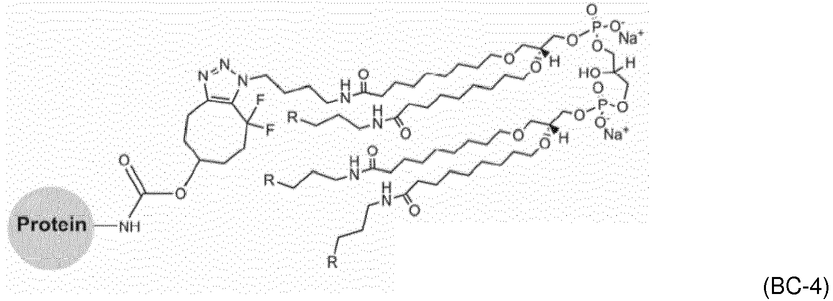 wherein: 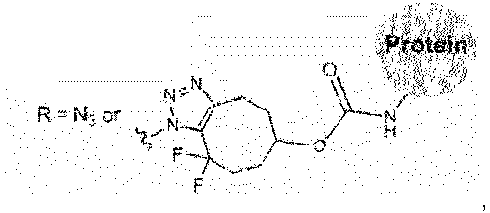 and 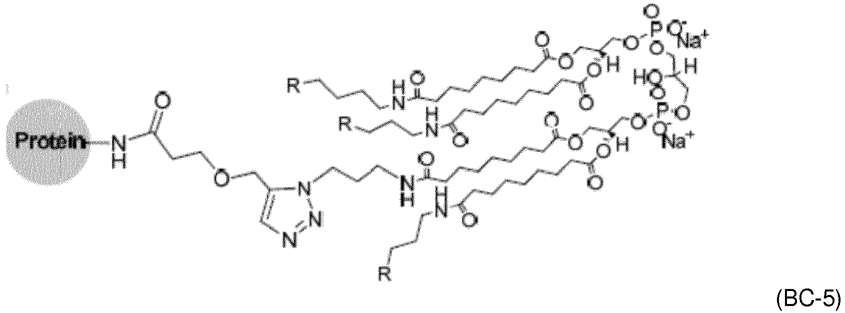 wherein: 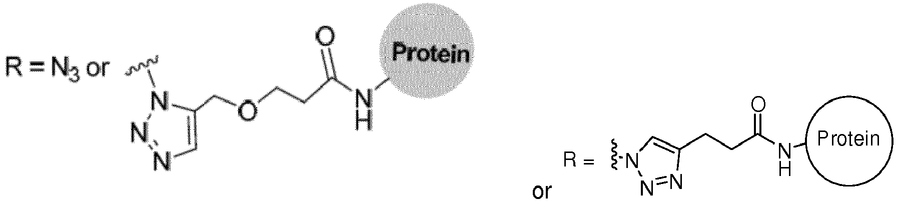 and, 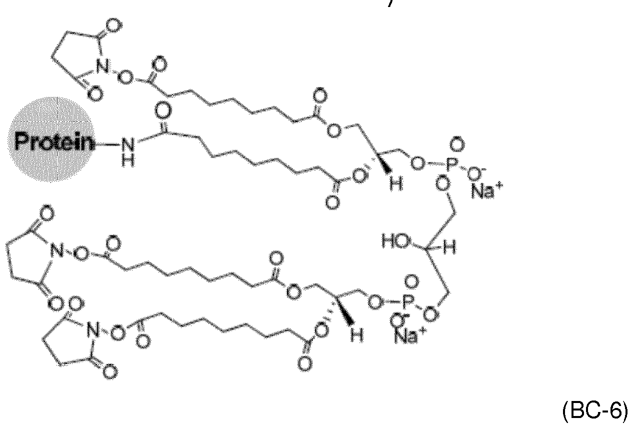 Importantly for BC-1 to BC-6 Na⁺ may be replaced with any other appropriate cation, such as K⁺ or Ca²⁺. The "protein" moiety of the present invention is selected from beta2-GPI and prothrombine.

Described herein (not part of the invention) is a conjugate of formula (BC-7): 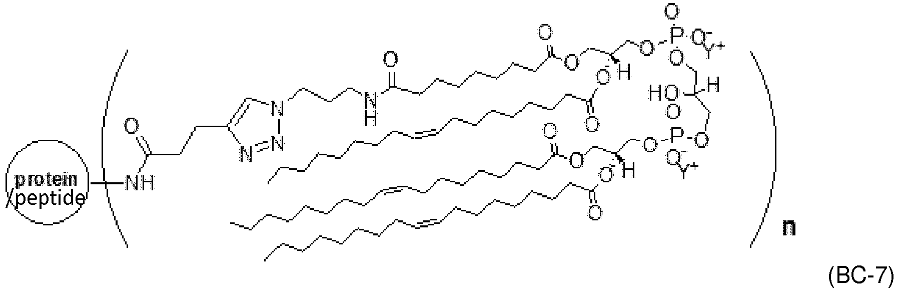 wherein n is an integer and may be in the range of 1-20, such as 5-15, such as preferably 10-13. The protein/peptide moiety may be any of the proteins and peptides including beta-2 glycoprotein I and/or prothrombine. When the protein/peptide moiety is prothrombine, n is preferably 12-13. When the protein moiety is beta-2 glycoprotein I, n is preferably 10-11. Y⁺ may be any appropriate cation. Preferably Y⁺ may be Na⁺, K⁺, or ½Ca²⁺.

It is noted that the bioconjugate (BC-7) may be obtained by the "click" coupling of cardiolipin analogue (CL-5) and protein intermediate (int-3). This is analogous to the reaction of Scheme II below.

Compounds of formula BC-7 have been shown to be surprisingly specific in the detection of autoimmune anti-bodies and stable in the studies described herein, as compared to known conjugates of the prior art. Without being bound to theory it is believed that the elongated linker and oxidized state of the phospholipid may be responsible to the improvements detected.

Described herein (not part of the invention) is a conjugate of formula (BC-8): 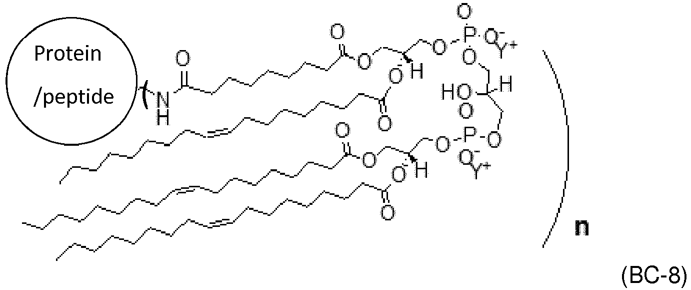 wherein n is an integer and may be in the range of 1-20, such as 5-15, such as preferably 10-13. The protein/peptide moiety may be any of the proteins and peptides including beta-2 glycoprotein I and/or prothrombine. When the protein/peptide moiety is prothrombine, n is preferably 12-13. When the protein moiety is beta-2 glycoprotein I, n is preferably 10-11. Y⁺ may be any appropriate cation. Preferably Y⁺ may be Na⁺, K⁺, or ½Ca²⁺.

Another aspect of the present invention is a method of detecting an autoimmune antibody using a conjugate of formula (BC-1) or (BC-2) in an enzyme-linked immunosorbent assay (ELISA).

Described herein (not part of the invention) are cardiolipin analogues (CL-1) to (CL-4) for conjugation with a peptide or a protein selected from the group consisting of: 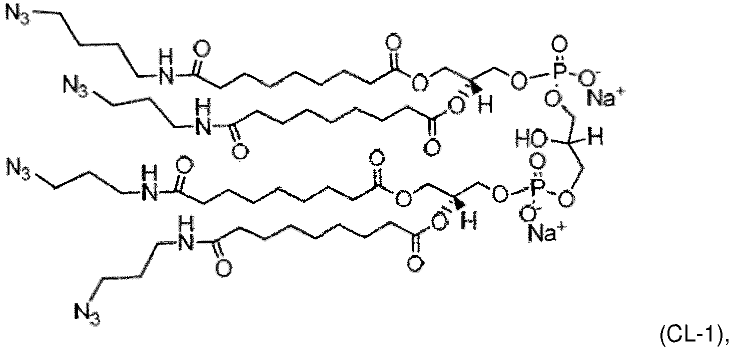 and 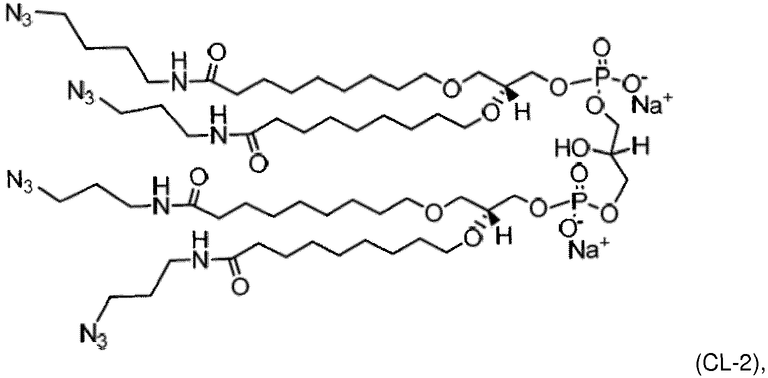 and 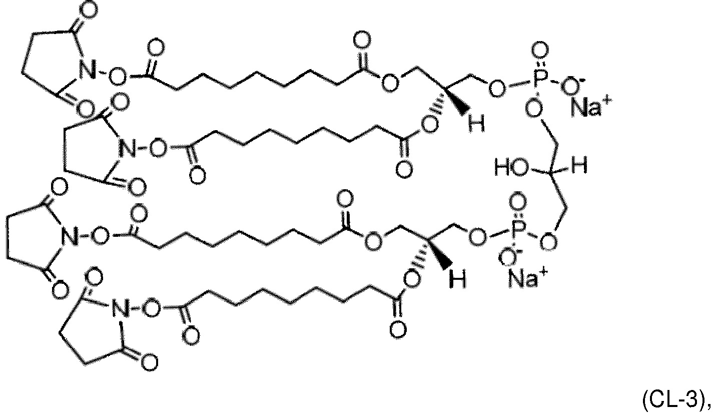 and 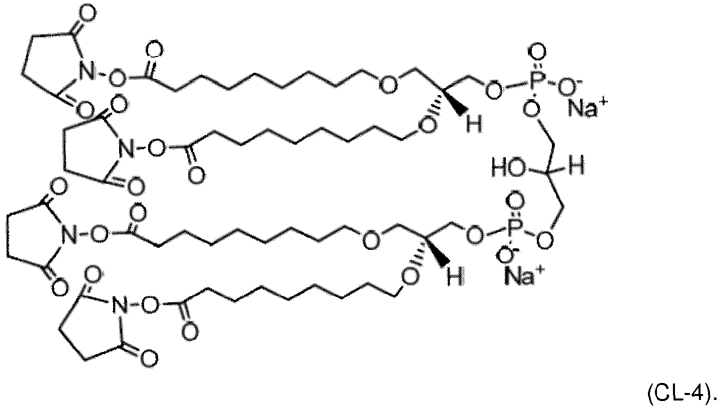 Importantly, for CL-1 to CL-4 Na⁺ may be replaced with any other appropriate cation, such as K⁺ or Ca²+.

Described herein (not part of the invention) is a cardiolipin analogue for conjugation with a peptide or protein is a compound selected from the group consisting of Formula (CL-5) and Formula (CL-6): 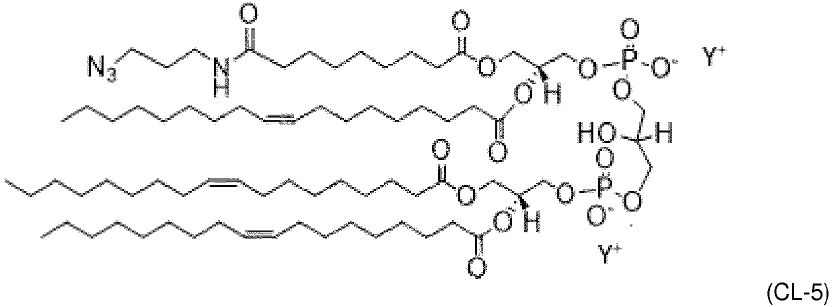 wherein Y⁺ may be any appropriate cation. Preferably Y⁺ may be Na⁺, K⁺, ½Ca²⁺. 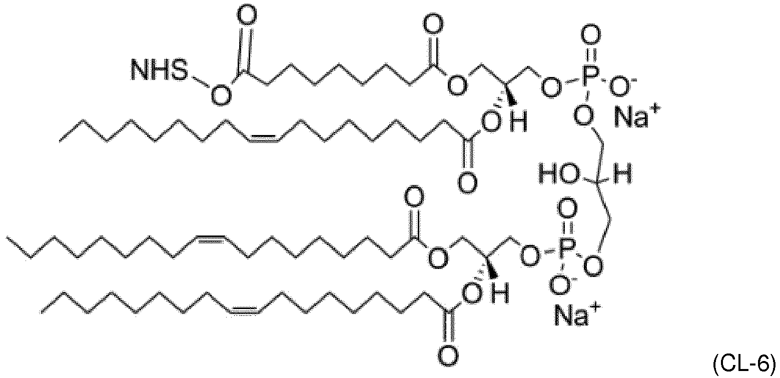 Wherein, NHS denotes N-Hydroxysuccinimide, and for for (CL-6) Na⁺ may be replaced with any other appropriate cation, such as K⁺ or ½Ca²⁺.

Described herein (not part of the invention) are peptide and protein derivatives selected from the following general formulae (int-1) to (int-3), which are useful for preparing the conjugates Z 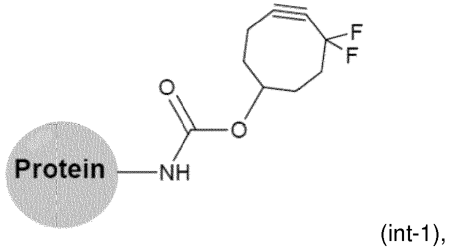 and 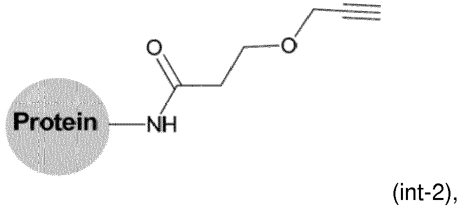 and

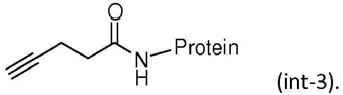

In a still another aspect of the present disclosure (not part of the invention) there is provided a method for preparing the phospholipid-peptide and phospholipid-protein conjugates, said method comprising reacting a protein derivative with a cardiolipin phospholipid in accordance with the following scheme I, Scheme II, or Scheme III:

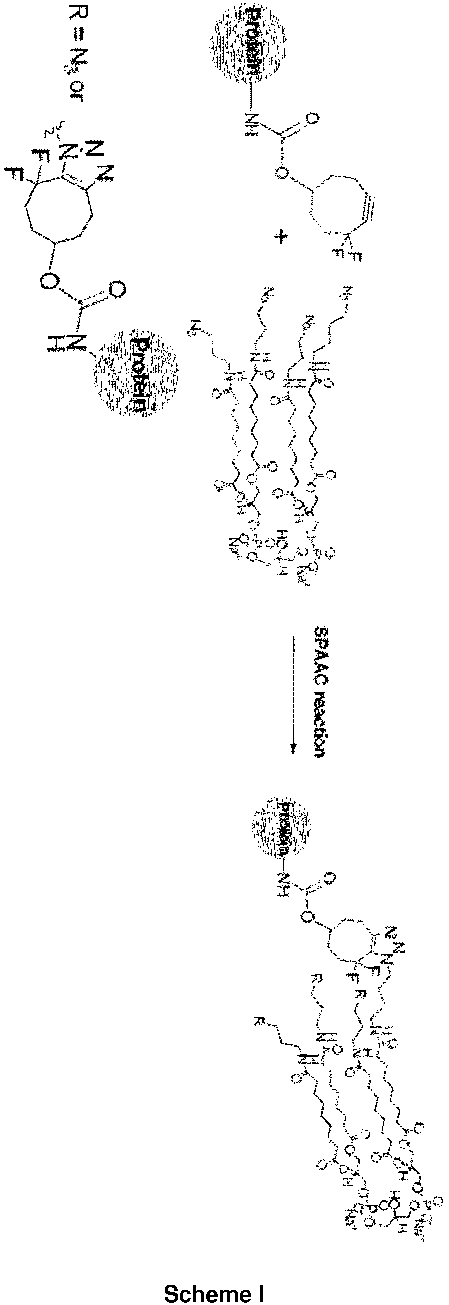

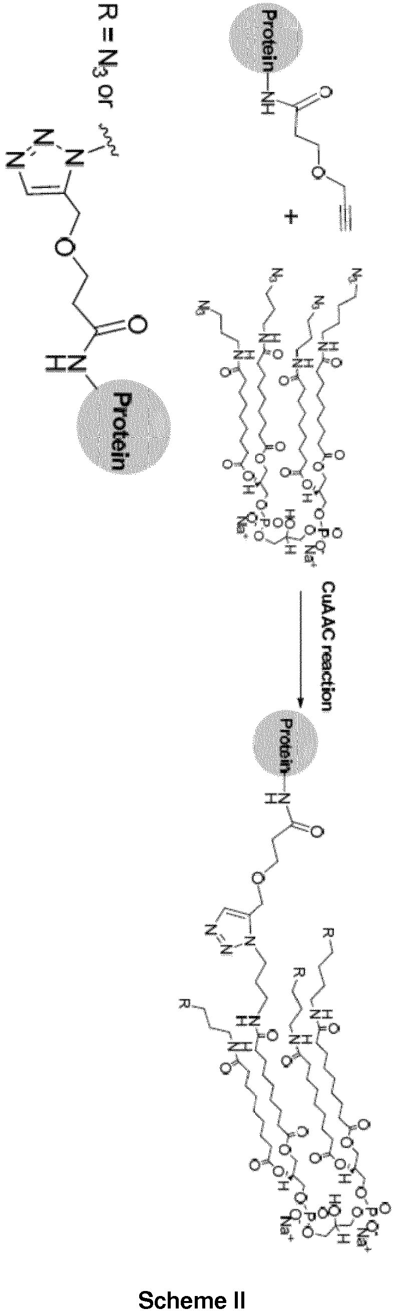

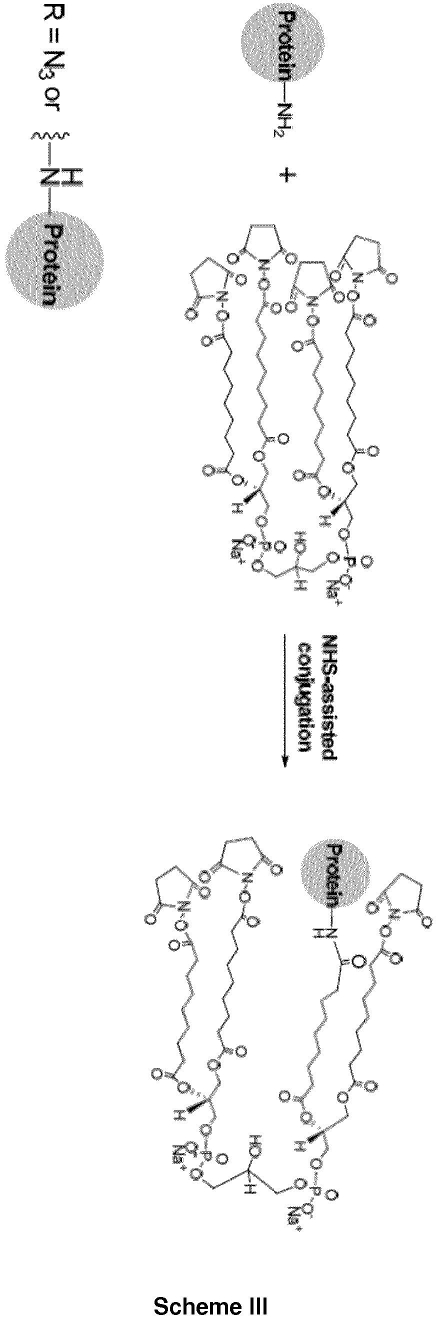

The "protein" moiety may be any of the proteins and peptides including beta2-GPI and prothrombine.

In still another aspect described herein there is provided an approach for attaching cardiolipin analogues (or lipoidal antigens comprising cardiolipin modified as described herein) to a peptide/protein (or solid support) while maintaining the antigenicity and specificity of the antigen for anti-lipoidal antibodies. Using methods described herein, it is now possible to create cardiolipin-attachment molecule complexes. The ability to attach the immunogenic cardiolipin complex in this manner allows it to be used in non-solution-based immunoassays, such as ELISAs and immunoassay devices.

### BRIEF DESCRIPTION OF THE DRAWINGS

**Figure 1** shows a synthethic strategy for preparation of novel bioconjugates by SPAAC click chemistry. (b) Detecting specific antibodies by the novel phospholipid-peptide and phospholipid-protein bioconjugates in homogeneous and 2D array formats.
**Figure 2** shows a general strategy for synthesis of novel cardiolipin analogues with improved chemical stability.
**Figure 3** shows a scheme of enzyme-linked immunosorbent assay (ELISA) for detection of autoimmune antibodies against antigens.
**Figure 4** shows comparative performance of phospholipid antigens in ELISA detection of autoimmune antibodies.

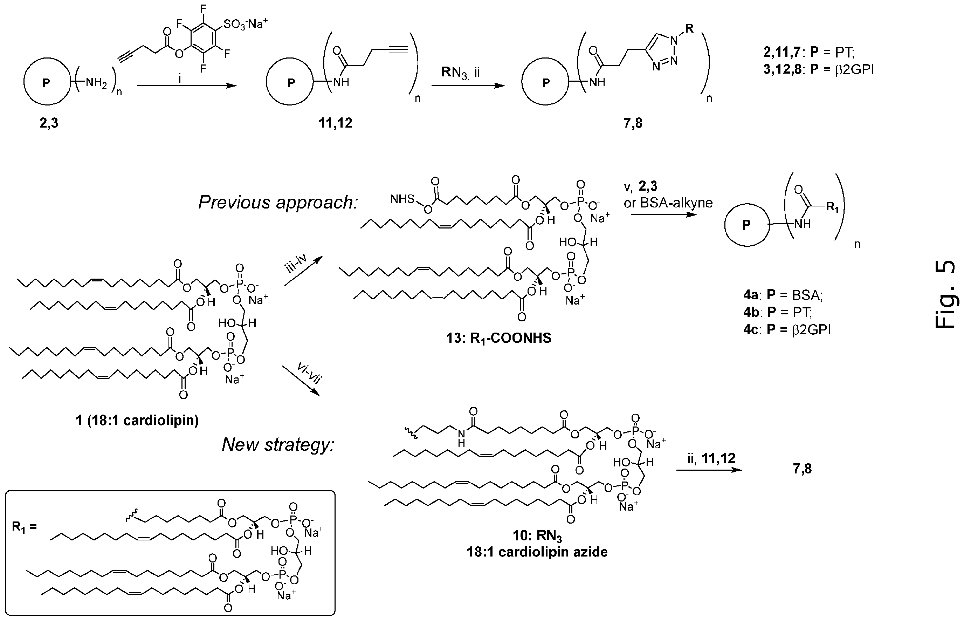

**Figure 5** shows synthetic strategies for cardiolipin-beta-2GPI and cardiolipin-PT complexes, particularly compounds 7 and 8 therein. Reagents and conditions: (i) 0.1 M bicarbonate buffer-DMSO 9:1, 4°C, 12 h; (ii) corresponding azide, CuSO4:TBTA 1:1.1, ascorbic acid, 1× PBS-DMSO-t-BuOH 3:2:0.1, v/v/v; (iii) KMnO4, NalO4, t-BuOH-H2O 9:1, v/v, rt, 12 h; (iv) succinimide ester, N,N'-diisopropylcarbodiimide, DMSO, rt, 12 h; (v) proteins 2,3,8, 0.1 M bicarbonate buffer-DMSO 9:1, v/v, rt, 12 h; (vi) KMnO4, NalO4, t-BuOH-H2O 9:1, v/v, 45 °C, 1 h, microwave reactor; (vii) succinimide ester, N,N'-diisopropylcarbodiimide, DMSO, rt, 5 h; □ 3-azidopropan-1-amine, rt, 12 h.
**Figure 6A and 6B** shows results of IgG ELISA assay [absorbance for grouped patients (6B) and healthy controls (6A) across antigens], obtained using compounds 7-8 of Figure 5 and controls. Negative and positive controls (HNP and HCL, Immunovision) are shown as a "x" and a star, respectively. Conjugates 5 and 6 in Fig 6A corresponds to compounds 6 and 7 respectively of Scheme VI herein.
**Figure 7** shows data plot for observed correlations between aPLPs and clinical parameters for disease stated patients (SUH cohort). Students's t-test indicated a correlation between Smith positivity and elevated aPLP titers using exclusively antigen **8** of Figure 5.
**Figure 8** shows linear regression plot for observed correlations between aPLPs and clinical parameters for disease stated patients (SUH cohort). Ordinary least squares (OLS) analysis was performed using Stata. The difference in aPLP titers for Smith negative and Smith positive groups was found to be statistically significant with p value of 0.002 for antigen **8** of Figure 5.

### DETAILED DESCRIPTION

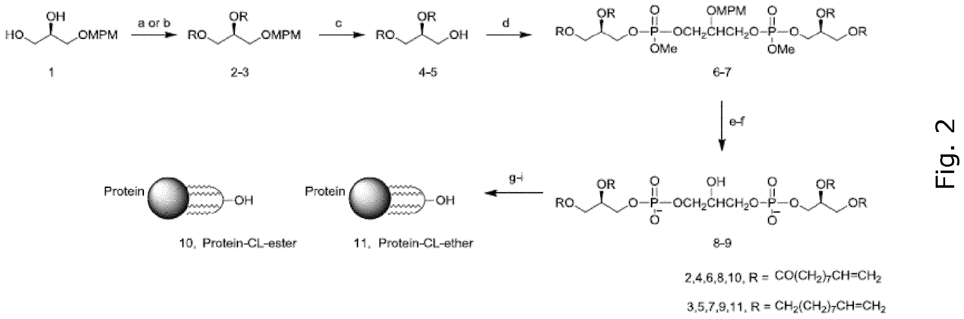

Herein, the bioconjugates in focus will be cardiolipin and phosphatidylserine attached to beta2-glycoprotein I (beta2-GPI), prothrombin and their short immunogenic fragments (peptide with a general sequence An, where A is an amino acid and n is between 10 and 50). Synthetic cardiolipin analogues with increased chemical stability are prepared in accordance with Figure 2, and then applied in bioconjugation with beta2-glycoprotein I or its short fragment.

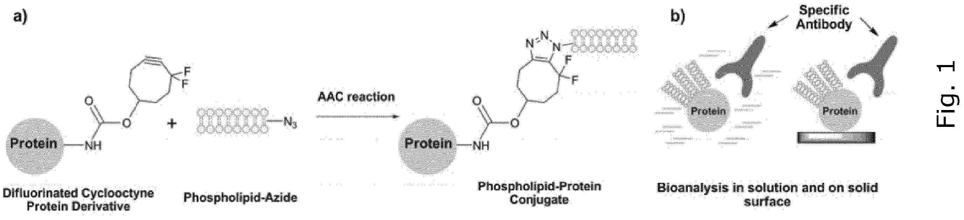

Next, chemical modification of the novel azide derivatives of cardiolipin and phosphotidylserine will be linked to the terminal alkyne or difluorinated cyclooctyne moiety of the corresponding proteins under CuAAC and SPAAC reaction conditions (Figure 1a). In preparation of phospholipid-peptide and phospholipid-protein conjugates for diagnostics and studies of autoimmune disorders, convenient bioconjugation strategies are on high demand. Among other methods available to date, two variants of azide-alkyne cycloaddition, namely a copper-catalyzed and strain-promoted click reactions (CuAAC and SPAAC, respectively), were recently demonstrated as promising synthetic approaches giving stable 1,2,3-triazole products both in vitro and in vivo (H. C. Hang, J. P. Wilson, G. Charron. Acc. Chem. Res. 2011, 44, 699, and J. A. Precher, C. R. Bertozzy. Nat. Chem. Biol. 2005, 1, 13). Thus, azide- and alkyne-functionalized fatty acid and lipid reporters were successfully attached to cells by CuAAC reaction providing powerful tools for monitoring lipid trafficking and metabolism (H. C. Hang, J. P. Wilson, G. Charron. Acc. Chem. Res. 2011, 44, 699). Furthermore, bioconjugation of biotin with several lipid reporters was achieved in high yields using CuAAC protocol (J. A. Precher, C. R. Bertozzy. Nat. Chem. Biol. 2005, 1, 13). The disadvantage of CuAAC reaction is a high toxicity of copper required for the reaction. This limits CuAAC bioconjugation strategy to exclusively in vitro conditions. In turn, SPAAC approach was extensively utilized for bioconjugation in vivo giving an opportunity to monitor cellular processes and metabolic pathways of whole organisms, although with a main focus on proteins and glycans (J. A. Precher, C. R. Bertozzy. Nat. Chem. Biol. 2005, 1, 13).

In order to perform bioconjugation by SPAAC approach, azide and cyclooctyne functionalities need to be attached to biomolecules (Figure 1a). This can be done using commercially available reagents and following protocols described in literature. (M. G. Paulick, A. R. Wise, M. B. Forstner, J. T. Groves, C. R. Bertozzi. J. Am. Chem. Soc. 2007, 129, 11543) Providing high yields of the desired bioconjugates in mild nontoxic conditions, SPAAC click chemistry seems to be an ideal method of choice in preparation of novel phospholipid-protein conjugates which can be applied for bioanalysis both in vitro and in vivo.

Current bioanalysis can be performed in several formats requiring preparation of different reagents and application of conditions adapted for exactly this method. One promising method for detection of specific antibodies is a homogeneous ("all-in-solution") analysis which is a simple and rapid technique (M. Chassignol, Y. Aubert, V. Roig, U. Asseline. Nucleos. Nucleot. Nucl. Acids 2007, 26, 1669). The homogeneous analysis can be performed using, for example, fluorescent labeling of target biomolecules and following their interaction by monitoring fluorescence signal. Immobilization on 2D array is another promising method of bioanalysis, which is especially powerful for serodiagnostics of human disorders due to high sensitivity of this method and possibility of target antibody's type validation. Proteins, peptides and DNA can be immobilized on 2D arrays following previously described methods (A. M. Rouquette, C. Desgruelles. Lupus 2006, 15, 403, and references cited therein, and A. Castro. H. Wang. WO 2007/061793 A2, PCT/US2006/044572). However, in case of relatively small lipid molecules a protein cargo is required for attachment to the array which protects lipid from interactions with surface and, therefore, prevents loss of its immunogenic activity. Previously, nitrocellulose strips containing natural cardiolipin attached to BSA and KLH proteins were prepared and further applied in serodiagnostic studies (A. Castro. H. Wang. WO 2007/061793 A2, PCT/US2006/044572). Providing high immunogenic activity right after preparation, the resulting conjugates were rapidly degraded under array storage by unknown mechanism (This observation has been recently made at the Central Research Institute of Epidemiology, Moscow, Russia, while repeating synthetic procedures described in WO 2007/061793 A2). Therefore, a perspective direction in this area is a preparation of synthetic cardiolipin analogues with increased chemical stability compared to natural prototypes followed by attachment to proteins/peptides and immobilization (J. S. Yadav et al. Tetrahedron Lett. 1996, 37, 6603 and K. Kasireddy et al. Bioorg. Chem. 2005, 33, 345).

### EXAMPLES

The invention relates to compounds (BC-1) and (BC-2) as defined in the appended claims.

### EXAMPLE 1 - Synthetic approach to protein phospholipid conjugates of formula 10-11 of Figure 2

The reagents and conditions for the synthesis according to Figure 2 are:
a) 9-decenoic acid, DCC, DMAP, DCM, 0 °C; b) NaH, 10-bromo-1 -decene, DMF, 0 °C to rt; c) DDQ, DCM, H₂O, 0 °C; d) MeOPCl₂, DIPEA, THF, -78 °C; HOCH₂CH(OMPM)CH₂OH, - 78 °C to rt; 30% aq. H2O2, DCM, rt; e) DDQ, DCM, H₂O, 0 °C; f) Nal, 2-butanone, 80 °C; g) NalO₄, KMnO4, tBuOH, H₂O, rt; h) DIC, NHS, rt; i) Protein, buffer 12t, rt.

The anchoring of the analogues (**8-9**) of Figure 2 to protein or peptide may be achieved by attachment of fatty acid residues with terminal double bonds oxidized with NalO4-KMnO4, and afterwards NHS-assisted coupling to the protein. Alternatively, CuAAC and SPAAC approaches using azide-derivatives of the phospholipids **8-9** and the alkyne-labeled proteins can be performed. For these conjugations the above mentioned step h) is as follows:

### SPACC conjugation

h) DIC, NHS, 12t, rt; i) 3-azidopropan-1-amine, tBuOH, Et₃N, 3t, rt; j) Protein/peptide cyclooctyne derivative, 3-24 t,rt.

### CuAAC conjugation

h) DIC, NHS, 12t, rt; i) 3-azidopropan-1-amine, tBuOH, Et₃N, 3t, rt; j) Protein/peptide alkyne derivative, Cu-TBTA, ascorbic acid, 3-24 t,rt.

### EXAMPLE 2 - Flourescent labeling and ELISA assays

Documented immunogenity of synthetic lipids accompanied by varified chemical structure and purity of the phospholipid-peptide and phospholipid-protein conjugates has provided novel scientifically and clinically important information on the phospholipid-antibody binding process.

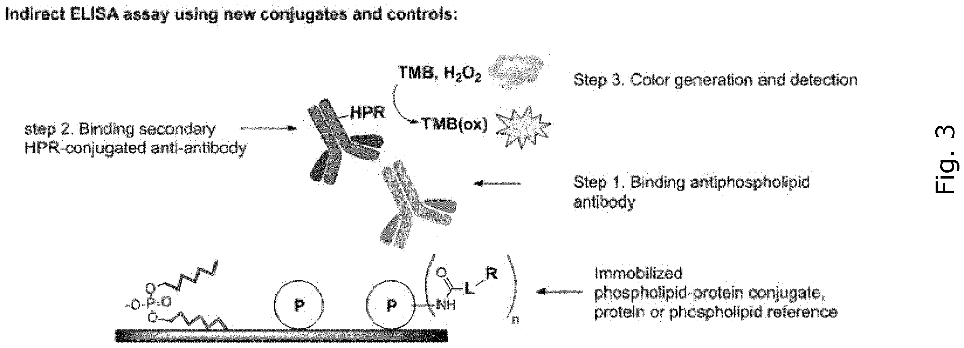

These key studies can be performed in two tests. First, we will test the phospholipid-peptide and phospholipid-protein constructs of the present invention in a homogeneous assay using fluorescent labeling. We will also use enzyme-linked immunosorbent assay (ELISA) for targeting specific autoantibodies by the prepared bioconjugates. Herein, a secondary HPR-labeled antibody will signal interaction between the synthetic phospholipid-peptide and phospholipid-protein antigens and the antibodies in serum samples of patients diagnosed with APS and other autoimmune diseases (see e.g. Figure 1b and Figure 3).

### EXAMPLE 3 - Synthesis and stability studies of protein-cardiolipin bioconiuaates

**Reagents and solvents.** Cardiolipin sodium salt from bovine heart (Sigma-Aldrich); synthetic cardiolipin (Avanti Polar Lipids); phosphatidylcholine from egg (PC L-α-isomer; Avanti Polar Lipids); phosphatidylcholine synthetic (18:1 Δ9-cis-isomer; Avanti Polar Lipids); KMnO₄, NalO₄, Na₂SO₃, albumin from bovine serum (BSA), lysozime, myoglobin, hemocyanin from Megathura crenula (KLH), *N*-hydroxysuccinimide (NHS; all from Sigma-Aldrich), β2-glycoprotein I human, non-recombinant (Diarect antigens), *N,N'-*diisopropylcarbodiimide (DIC; Fluka), DMSO (Sigma-Aldrich), t-butanol (Sigma-Aldrich), sodium dodecyl sulfate (SDS; Sigma-Aldrich).

**Methods.** Reactions were carried out in 1.5 mL plastic eppendorf tubes; mixing was performed using Eppendorf thermomixer 5436 shaker, evaporation was performed using rotary centrifuge (HETOVAC VR-1).

**Analysis.** Lipid molecules were analyzed using MALDI HRMS (Bruker microflex). Protein-lipid conjugates were analyzed by LC-MS (Bruker).

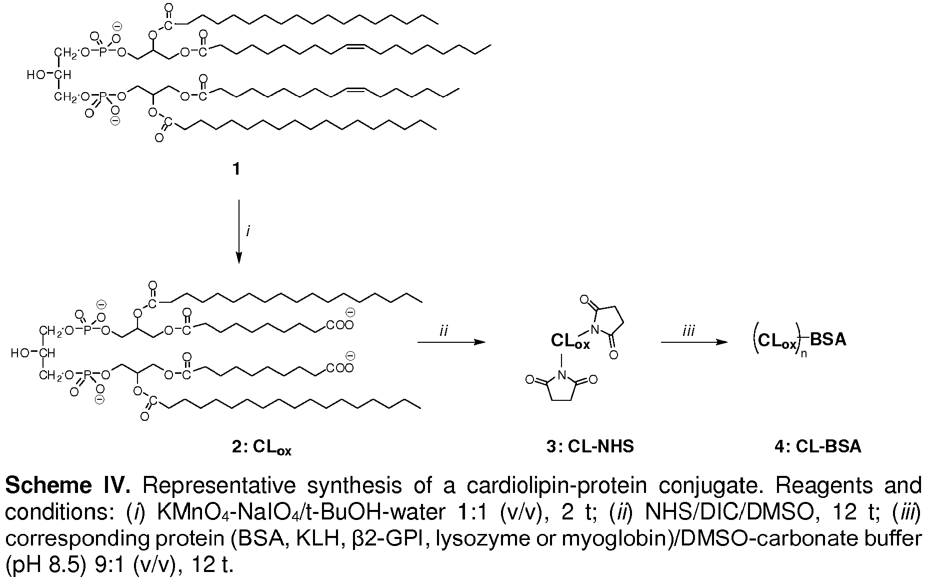

### Synthesis of oxidized cardiolipin Clₒₓ (2) - step i

To solution of cardiolipin 1 (20 mg; natural (n) or synthetic (s)) in 2 mL t-BuOH 20 mg NaHCO₃ in 100 mkl MQ water was added and mixed for 5 min. Afterwards 12 mg NalO₄ in 120 mkl water, 15 mg KMnO₄ in 400 mkl water and 500 mkl t-BuOH were subsequently added. The mixture was kept at room temperature for 2 hours, then 150 mg Na₂SO₃ was added and mixed for additional 10 min. The resulting mixture was centrifuged at 12000 rpm for 5 min, and upper phase was taken with a pipette. A t-BuOH phase was washed with 5% aq. HCI in saturated aq. NaCl solution, evaporated to dryness on a speedvac and redissolved in DMSO.

In parallel, oxidation of cardiolipin was performed as described above in presence of 16 mg of phosphatidylcholine (Scheme V; PC natural (n) or PC synthetic (s)), which is known to prevent oxidation of cardiolipin upon exposure to air. Thus, the oxidation experiments gave four lipid reagents for further attachment to protein cargos: CLox(n), CLox(s), CLox(n)-PCox(n) and CLox(s)-PCox(s). The oxidation was confirmed by HRMS MALDI spectra.

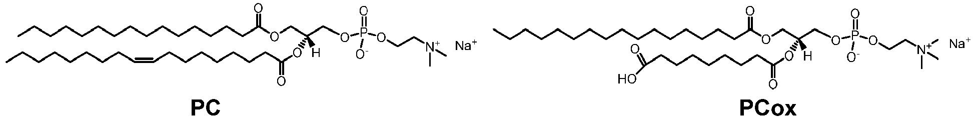

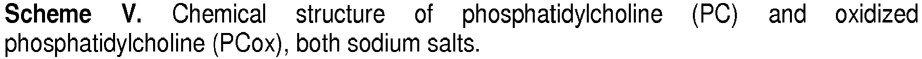

### Synthesis of activated ester of cardiolipin CL-NHS (3) - step ii

To a solution of oxidized lipid reagent 2 (10 mg of cardiolipin) in 1 mL DMSO 5 mg of NHS and 10 mkl of *N,N'*-diisopropylcarbodiimide were added. Reaction mixture was stirred till the lipids were completely dissolved (approx. 2 hours). The reaction was left at room temperature overnight. Resulting solution was used in the next step without purification. Storage of CL-NHS: at -70 °C.

### Synthesis of protein-cardiolipin conjugates (CL-protein)

To a solution of 1 mg of protein in 100 mkl of 0.2 M carbonate buffer (pH 8.5) 875 mkl DMSO and 15 mkl of a solution of CL-NHS 3 (10 mg/mL) were added. Reaction was mixed for 20 min and then left overnight. Resulting reagent is useful for diagnostics according to literature without purification. We evaluated stability of the conjugates in the mixtures described below after 4 months of storage at +4 °C and after 1 year storage at -20 °C by LC-MS (Table 1). Natural lipid components were previously applied by other groups. As one can see, these reagents are rapidly degraded during storage, whereas using synthetic lipids and developed storage conditions (especially 3 and 4) the conjugates were stable up to 1 year at -20 °C without need in de-aeration. The best results of stability to degradation upon storage were shown by the novel conjugates 8, 10, 16 and 18 prepared herein. Stabilities are given in table 1 below.

**Table 1. Composition and stability of the lipid-protein conjugates prepared in this study. ***

| Reagent Package No. | | Lipid component | Protein component | Origin of lipid sample | Stability, % | | | | | | | |
|---|---|---|---|---|---|---|---|---|---|---|---|---|
| | | | | | 4 months | | | | 1 year | | | |
| | | | | | Storage mixture no: | | | | | | | |
| | | | | | *1* | *2* | *3* | *4* | *1* | *2* | *3* | *4* |
| 1 | Plastic vial | CL(n) | Bovine serum albumin (BSA) | Natural | 40 | 40 | 50 | 53 | 32 | 30 | 42 | 43 |
| 2 | Plastic vial | CL(s) | | Synthetic | 66 | 68 | 70 | 66 | 64 | 58 | 68 | 66 |
| 3 | Plastic vial | CL(n)-PC(n) | | Natural | 34 | 35 | 54 | 57 | 31 | 33 | 42 | 47 |
| 4 | Plastic vial | CL(s)-PC(s) | | Synthetic | 64 | 65 | 60 | 78 | 54 | 50 | 64 | 68 |
| 5 | Plastic vial | CL(n) | Hemocyanin | Natural | 32 | 37 | 52 | 53 | 37 | 30 | 45 | 50 |
| 6 | Plastic vial | CL(s) | | Synthetic | 40 | 38 | 73 | 78 | 38 | 42 | 85 | 79 |
| 7 | Plastic vial | CL(n)-PC(n) | | Natural | 38 | 40 | 44 | 54 | 38 | 40 | 44 | 54 |
| 8 | Plastic vial | CL(s)-PC(s) | | Synthetic | 44 | 50 | 65 | 84 | 44 | 50 | 65 | 84 |
| 7 | Plastic vial | CL(n) | Apolipoprotein H, human | Natural | 45 | 44 | 50 | 56 | 46 | 44 | 53 | 54 |
| 8 | Plastic vial | CL(s) | | Synthetic | 65 | 60 | 75 | 84 | 65 | 60 | 77 | 89 |
| 9 | Plastic vial | CL(n)-PC(n) | | Natural | 55 | 59 | 46 | 57 | 50 | 49 | 48 | 60 |
| 10 | | CL(s)-PC(s) | | Synthetic | 70 | 64 | 86 | 95 | 64 | 66 | 90 | 92 |
| 11 | Plastic vial | CL(n) | Lysozime | Natural | 34 | 31 | 56 | 45 | 34 | 22 | 36 | 40 |
| 12 | Plastic vial | CL(s) | | Synthetic | 45 | 61 | 74 | 45 | 44 | 45 | 70 | 56 |
| 13 | Plastic vial | CL(n)-PC(n) | | Natural | 32 | 36 | 65 | 32 | 30 | 30 | 66 | 45 |
| 14 | Plastic vial | CL(s)-PC(s) | | Synthetic | 55 | 68 | 74 | 55 | 50 | 47 | 70 | 58 |
| 15 | Plastic vial | CL(n) | Myoglobin | Natural | 34 | 30 | 45 | 42 | 33 | 45 | 67 | 70 |
| 16 | Plastic vial | CL(s) | | Synthetic | 60 | 49 | 90 | 95 | 52 | 45 | **88** | 94 |
| 17 | Plastic vial | CL(n)-PC(n) | | Natural | 40 | 39 | 51 | 50 | 44 | 34 | 78 | 75 |
| 18 | Plastic vial | CL(s)-PC(s) | | Synthetic | 63 | 46 | 88 | 92 | 50 | 41 | 80 | 93 |

| | | | | | | | | | | | | |
|---|---|---|---|---|---|---|---|---|---|---|---|---|
| * Stability was evaluated as a ratio of corresponding LC peak area of the conjugate with a right averaged mass to the one of degraded products. in %. | | | | | | | | | | | | |

### Storage and analysis of cardiolipin-protein conjugates

The following mixtures of conjugates were put to storage at +4 °C and -20 °C:
1. Solution of conjugate 1-18 in DMSO-0.2 M carbonate buffer (concentration - 10 mg/mL).
2. Solution of conjugate 1-18 in DMSO-0.2 M carbonate buffer (concentration - 10 mg/mL) with additional 1-thioglycerine (final concentration - 1mM).
3. Solution of 1-18 in DMSO-0.2 M carbonate buffer (concentration - 10 mg/mL) with additional 1-thioglycerine (final concentration - 5mM).
4. Solution of 1-18 in DMSO-0.2 M carbonate buffer (concentration - 10 mg/mL) with additional 1-thioglycerine (final concentration - 5mM) and SDS (final concentration - 0.01%).

Results are seen in table 1 above.

### EXAMPLE 4 - Synthetic approach to protein phospholipid conjugates using triazole-amide linkers

In this example a new CuAAC click approach for the attachment of synthetic PE (phosphoethanolamine) antigen to clinically relevant β2GPI and PT is demonstrated. In spite of structural differences, both CL and PE are immunogenic and proved to be useful in diagnostics of anti-phospholipids (aPLs). The click procedure has advantages of high yields and purity of the products with improved chemical stability compared to oxidized phospholipids. Moreover, the new procedure can be directly employed for similar quantities of different phospholipids and proteins. In order to compare with previously reported analogues, a PE-BSA conjugate was prepared as well (Scheme VI below). 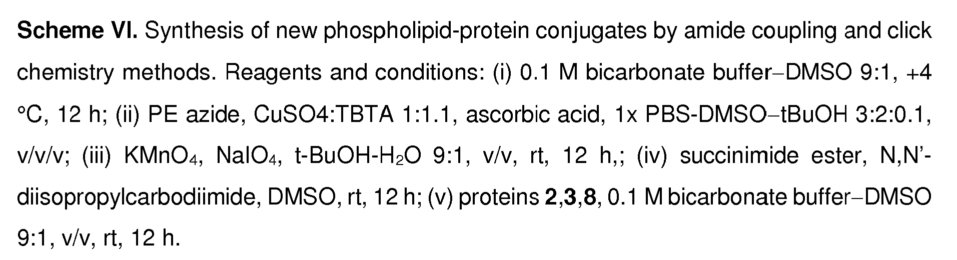 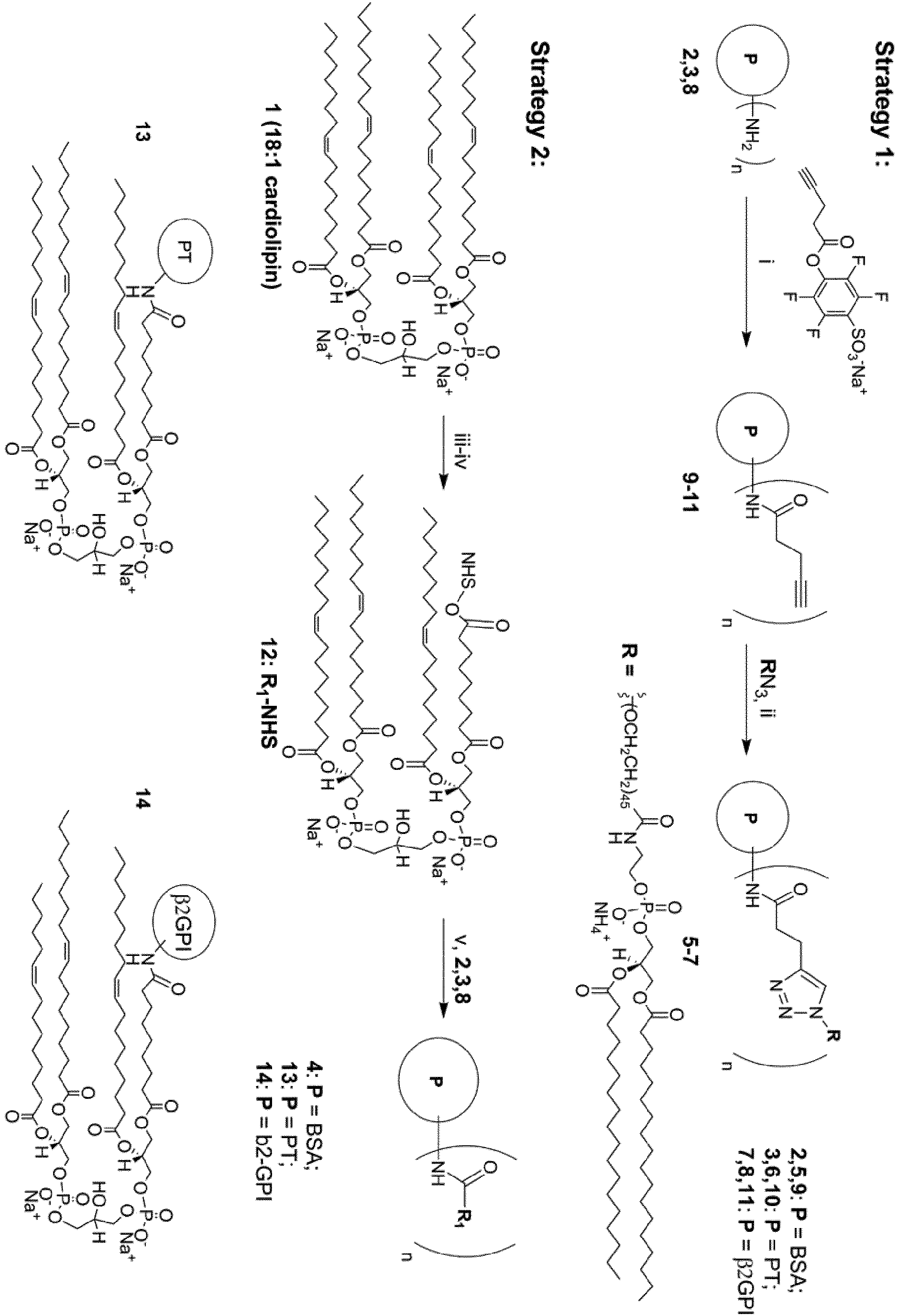 First, we functionalized each protein with water soluble alkyne activated ester alkyne group and then subjected it to CuAAC click reaction with commercially available PE azide in a molar ratio 1:25. After simple precipitation, we obtained the desired conjugated 5-7 in good yield and purity (Scheme 1; yields ≥ 80%; full conversion as determined by gel electrophoresis)The present inventor has demonstrated CuAAC chemistry as an improved method for preparation of novel phospholipid-protein conjugates with a high potential for the diagnostics of autoimmune diseases. The resulting phospholipid-protein conjugates show high binding affinity and specificity for the autoimmune antibodies.

Figure 3 shows a representative scheme of enzyme-linked immunosorbent assay (ELISA) for detection of autoimmune antibodies against antigens of the present invention: phospholipids, proteins and phospholipid-protein conjugates. P = protein, L = linker, R = phospholipid residue, TMB = 3,3',5,5'-tetramethylbenzidine, HPR = horseradish peroxidase.

Conjugates and control antigens we used in the enzyme-linked immunosorbent assay (ELISA), applying series of disease-associated or human normal plasmas (HNP, n = 10; Figure 3, Table 2). The disease-associated samples contained high levels of antibodies against phospholipids (aPLs), α-β2-GPIs; control samples to assess cross-reactivity contained autoantibodies to single-stranded and double-stranded DNA.

**Table 2. Results of ELISA assay using diverse antigens: cardiolipin, prothrombin, β2-glycoprotein, phosphoethanolamine azide and synthetic conjugates 5-7 of scheme VI.***

| Antigen | Absorbance at 450 nm: | | | | |
|---|---|---|---|---|---|
| | Analyte | | | | |
| | a-PL | a-β2GPI | a-ssDNA | a-dsDNA | HNP (n=10) |
| CL | 1.97 | 0.90 | 0.90 | 0.63 | 0.4 5 |
| β2GPI | 0.75 | 1.01 | 0.60 | 0.32 | 0.22 |
| CL:β2GPI^{§} | 0.98 | 0.80 | 0.55 | 0.61 | 0.52 |
| PT | 0.45 | 0.3 1 | 0.43 | 0.45 | 0.23 |
| BSA | 0.31 | 0.25 | 0.34 | 0.28 | 0.24 |
| PE azide | 1.03 | 0.91 | 0.80 | 0.54 | 0.33 |
| 5 (BSA-PE) | 1.44 | 0.56 | 1.44 | 1.21 | 0.43 |
| 6 (PT-PE) | 1.05 | 0.44 | 1.01 | 0.72 | 0.45 |
| 7 (β2GPI-PE) | 1.02 | 1.15 | 0.5 | 0.4 | 0.19 |

| | | | | | |
|---|---|---|---|---|---|
| * a-PL, a-β2GPI, a-ssDNA and a-dsDNA = human plasma tested highly positive against phospholipids; β2GPI, single-stranded and double-stranded DNA, respectively. HNP = human normal plasma; averaged absorbance for n patients is presented (Δ ± 0.20). § β2GPI (0.001%) was added to cardiolipin under blocking conditions resulting in non-covalent binding. CL = cardiolipin, PT = prothrombin. Each sample was measured in the duplicate with resulting deviation in absorbance Δ ± 0.20. | | | | | |

High cross-reactivity of aPLs with other antigens such as ssDNA and dsDNA is an obstacle for their utility in studies and diagnostics of autoimmune diseases. Improved specificity of aPL binding was achieved by covalent cross-linking of biologically complementary molecules such as PE with PT and PE with β2-GPI. The IgG ELISA experiments show that this has been achieved for PE-β2-GPI conjugate (absorbance 1.02 vs. 0.47-0.54 when incubated with APL, a-ssDNA and a-dsDNA, respectively) (Table 2).

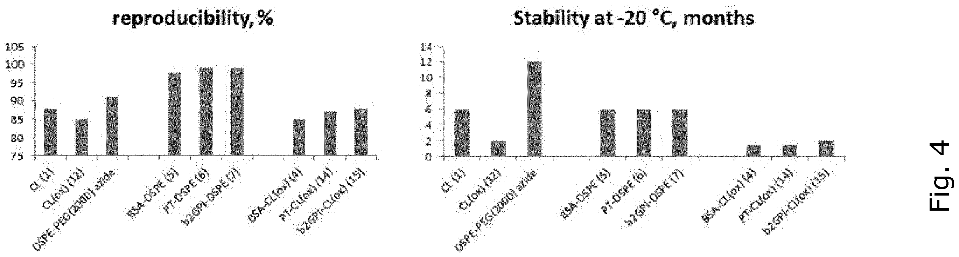

The reproducibility of ELISA tests and stability of antigens upon storage in solution at -20 °C was tested (Figure 4). The latter was done by TLC and gel electrophoresis. As appears from Figure 4 conjugates **5-7** showed superior reproducibility than individual phospholipids and oxidized cardiolipin conjugates (97-98% vs. 83-89%, respectively). Stability upon storage in solution was increased up to 6 months at -20 °C vs. 1.5-2 months for oxidized CL analogues. This implies that high purity and immunogenicity of the novel molecules has a positive effect on their diagnostic performance, which renders them applicable for studying various autoimmune conditions.

### EXAMPLE 5 - New strategy for the preparation of cardiolipin azide reagent and "click" conjugation to proteins

The rationale beyond new synthesis was to use synthetic, pure and well-characterized lipids and to decrease steric hindrance that arises upon bioconjugation to proteins. The latter was done using extended linker between the cardiolipin and azide moiety (see Figure 5).

### 5.1 Synthesis of cardiolipin-azide 10 of Figure 5

A solution of cardiolipin (CL) 1 (10 mg, 6.66 µmol) in 1.5 mL tBuOH 100 µL was mixed with NaHCO₃ (10 mg in 100 µL), NalO₄ (30 mg in 200 µL water) and KMnO₄ (10 mg in 200 µL water). The reaction was flashed with argon for 3 min and afterwards kept in a microwave reactor (15V) for 1 h at 45 °C. After the starting material was no longer detectable by TLC, the reaction was quenched by adding 150 mg Na₂SO₃. The mixture was afterwards acidified with 5% HCI to pH 3.0 and washed twice with t-BuOH. t-BuOH fraction was dried over Na₂SO₄ and evaporated in vacuo. Yield 86%; *R_{f}*0.37 (chloroform:methanol:water 3:1.5:0.2, v/v/v), HRMS-ESI *m*/*z:* 1406.84772 ([M + Na]+, C₇₂H₁₃₀Na₂O₁₉P₂ calcd 1406.84766).

To a solution of oxidized cardiolipin (5 mg) in 1 mL DMSO 4 mg succinimide ester in 20 µL DMSO and 8 µL N,N'-diisopropylcarbodiimide were added. After keeping the reaction for 5 h at room temperature TLC showed complete conversion of the starting material. Intermediate NHS-cardiolipin was further reacted with 3-azidopropan-1 -amine (1.1 eq., 0.35 mg) at room temperature in dark over 12 h. The product was used in further steps without purification. *R_{f}* 0.55 (chloroform:methanol:water 3:0.5:0.2, v/v/v), HRMS-ESI *m*/*z*: 1488.91209 ([M + Na]+, C₇₅H₁₃₆N₄Na₂O₁₈P₂ calcd 1488.91203).

### 5.2 Click reactions

Click reactions were performed as previously described herein. The products were initially purified by gel filtration using Zeba spin desalting columns (Life Technologies), and afterwards precipitated from cold acetone (-20 °C). The resulting conjugates were washed twice with cold acetone, dried in vacuo and analyzed by mass spectrometry and gel electrophoresis. Final yields of products based on the absorbance at 280 nm: 88% (6, scheme VI), 92% (7, scheme VI), 89% (7, Fig 5), 84% (8, Fig 5).

Chemical composition of product antigens 7-8 (Fig 5) was verified by gel electrophoresis and MALDI MS spectra. According to MS data, cardiolipin residues were attached to beta2GPI and PT, respectively. This is significantly higher than for a previous approach using direct coupling of NHS-cardiolipin derivative to proteins (1-2 residues), and might be a result of reduced steric hindrance of the cardiolipin reagent.

### EXAMPLE 6 - Superior specificity and stability of new molecules compared to controls

As mentioned above, low stability and high cross-reactivity are major issues of currently applied phospholipid antigens. As a result, detected antibodies are not specific to an autoimmune disease and the tests have low to no value for diagnostics. We prepared new antigens which contain covalently linked phospholipid and proteins which mimic biologically relevant autoimmune antigens. To validate their stability and specificity vs. controls, we carried out ELISA assays with well characterized human monoclonal antibodies toward HIV-1 antigens and healthy controls.

6.1. ELISA assays, reproducibility analysis and testing the stability of antigens were carried out as described before (Molecules 2015, 20, 10253-10263). Briefly, the ELISA assay includes two incubation steps followed by enzyme-promoted signal generation. First incubation involves binding of target antibodies within the sample to immobilized antigen on the surface of a microplate. At the second step, the bound antibody interacts with the secondary anti-antibody (e.g. anti-human IgG or IgM), covalently labeled with an enzyme such as horseradish peroxidase (HRP). In the presence of H₂O₂ HRP catalyzes conversion of non-colored organic substrate TMB to oxidized TMB(ox), which has a distinctive absorbance signal at 450 nm (A₄₅₀). Thus, by measuring A₄₅₀ one can quantify titers of particular antibodies towards immobilized antigen.

Initially, background signal of each sample was tested to be below A₄₅₀ 0.06 using non-coated microplates upon blocking at the same conditions as for the original experiment. To determine equilibrium time for the aPLP-antigen binding, pre-coated microplates were subjected to incubation with plasma samples in dilution 1:200 over a time course of 40 min - 3 hrs. Secondary incubation was always carried out for 1 h at room temperature using 1:35.000 dilution of corresponding HRP-conjugate. Results of this assay are presented in the Supporting Information.

Linear range for each antigen was determined by testing series of control dilutions (HNP, HCL in dilutions 1:50 to 1:2000). According to the results plasma dilutions 1:100 - 1:750 were within the linear range of assay for each antigen (R² > 0.97). For the controls and patient samples, weak positive (+/-) and positive (+) signal were determined as 2- and 3-fold signal, respectively, above the mean value for a healthy control cohort (n = 16).

### 6.2. Specificity assessment (compound numbers are as in scheme VI above or Figure 5 as noted for each compound)

The results of IgG class ELISA for novel antigens and controls obtained using monoclonal antibodies towards HIV-1 antigens and healthy sera samples are shown in Table 1. We observed the highest level of unspecific binding for non-covalent complexes of PLs and proteins **1**+**8** and **1**+**3** (scheme VI, mixed antigen control). In contrast, cross-linked conjugates (**6-7,** scheme VI) and (**7-8** Figure 5) showed only low to no non-specific binding in similar experiments (for example, Table 3, data for (**8,** Figure 5) compared to 1+8 and **1**+**3,** Scheme VI. Conjugates **6-7** (Scheme VI) and demonstrated a weak binding to anti-gp41, whereas binding to anti-p24 and B12 antibodies completely disappeared upon PL-protein conjugation. High reactivity on HIV-1 specific monoclonal antibodies and healthy control sera observed for mixed PLP controls could result from the high heterogeneity of non-covalent structures leading to unspecific interactions with polyclonal antibodies. In turn, cross-reactivity of phosphoethanolamine containing antigens 6-7(scheme VI) might be caused by the presence of PEG linker. Alternative linker design for the attachment of phosphoethanolamine to proteins is an objective of our on-going research and will be published in the near future. Finally, the most specific antigens **7-8** of Figure 5) were further applied in the studies of human samples described below.

**Table 3. Analysis of binding specificity for cross-linked antigens and controls.numbering as in Scheme VI unless otherwise stated.***

| Antigen nr./details* | Binding human monoclonal antibodies: | | | Response in healthy controls (% of patients) |
|---|---|---|---|---|
| | a-p24 | a-gp41 | B12 | |
| **6**/PE^{PEG}-PT | - | + | - | +/- (12%) |
| **7**/PE^{PEG}-β2GPI | - | +/- | - | + (6%), +/- (12%) |
| **7,** **Fig 5****)**/CL-PT | - | +/- | - | +/- (6%) |
| **8**, **Fig 5****)**/GL-β2GPI | - | - | - | - (100%) |
| **8**/β2GPI | - | +/- | - | +/- (25%) |
| **3**/PT | +/- | - | +/- | +/- (25%) |
| **1+8**/CL + -β2GPI | +/- | +/- | +/- | + (44%), +/- (25%) |
| **1+**3/CL + PT | +/- | +/- | + | + (25%), +/- (25%) |
| **8+RN₃**/PE ^{PEG} +02GPI | +/- | +/- | + | + (25%), +/- (12%) |
| **3+RN₃**/PE^{PEG}+β2GPI | +/- | +/- | + | + (25%), +/- (25%) |

| | | | | |
|---|---|---|---|---|
| * Weak positive (+/-) and positive (+) signal were defined as 2- and 3-fold absorbance signals, respectively, above the mean value for a healthy control group. PE^{PEG}, PT, CL, β2GPI are PEGylated phosphoethanolamine, prothrombin, cardiolipin and β2-glycoprotein I, respectively. Covalent cross-linking and non-covalent mixing of the antigens is defined as hyphen and plus, respectively. | | | | |

### 6.3. Reproducibility and chemical stability of antigens 7-8 of Figure 5 upon diverse storage conditions

Experiments were carried out as described herein and showed that ELISA assays using 7-8 of figure 5 were highly reproducible with absorbance values varying within a range of 0.5-0.8 of mean titer for each independent analysis. Antigens 7-8 and cardiolipin were also analyzed by gel electrophoresis, ELISA and MALDI MS after storage in dark glass vials at -20 °C - +4 °C, and being immobilized on microtiter plates at +4 °C. Antigens 7-8 were stable for up to 3 months at +4 °C in glass vials and on the surface of microtiter plates, and up to 1 year in glass vials at -20 °C. Remarkably, cardiolipin which is used in current kits degraded after approx. 3 weeks of storage at +4 °C in a glass vial or on the microtiter plate, and after 3 months of storage in a vial at -20 °C.

### EXAMPLE 7 - Autoantibody detection in patient samples using new antigens

The initial ELISA assay was done manually using microtiter plates coated with antigens 6-**7** of Scheme VI and **7-8** of Figure 5 or control antigens. First, pre-coated microplates were analyzed using commercially available polyclonal controls (human normal plasma HNP and human plasma containing antibodies to cardiolipin, HCL, Immunovision). Plasma titration experiments showed low response of IgM antibodies to the dilution, whereas IgG antibodies showed reduced signal upon dilution with a high degree of linearity (R2 > 0.95; Supporting Information). Therefore, in this work we focused on the IgG assay. In addition, repeatability of assays and PLP stability were studied as described before and showed superior levels compared to controls in agreement with our previous data.

Next, studies of the SUH cohort (27 patients diagnosed with pediatric SLE) under the developed ELISA conditions identified five and seven aPLP positive patients (19% and 26%) when antigens 7 and 8 (Fig 5) were applied. This was in contrast with nine and three positive patients (33% and 11%) detected using beta2GPI and mixed antigen cardiolipin+beta2GPI, respectively. Robustness and clinical relevance of the new antigens was further evaluated by the analysis of blood samples from adult patients obtained from Odense University Hospital, Denmark (OUH). The assay was performed using similar plate coating, incubation conditions, controls and cut-off values as for SUH cohort, although in a fully automated regimen. The results were compared to commercial anti-cardiolipin and anti-beta2GPI tests (Euro Diagnostica), which were performed for OUH patients under similar conditions.

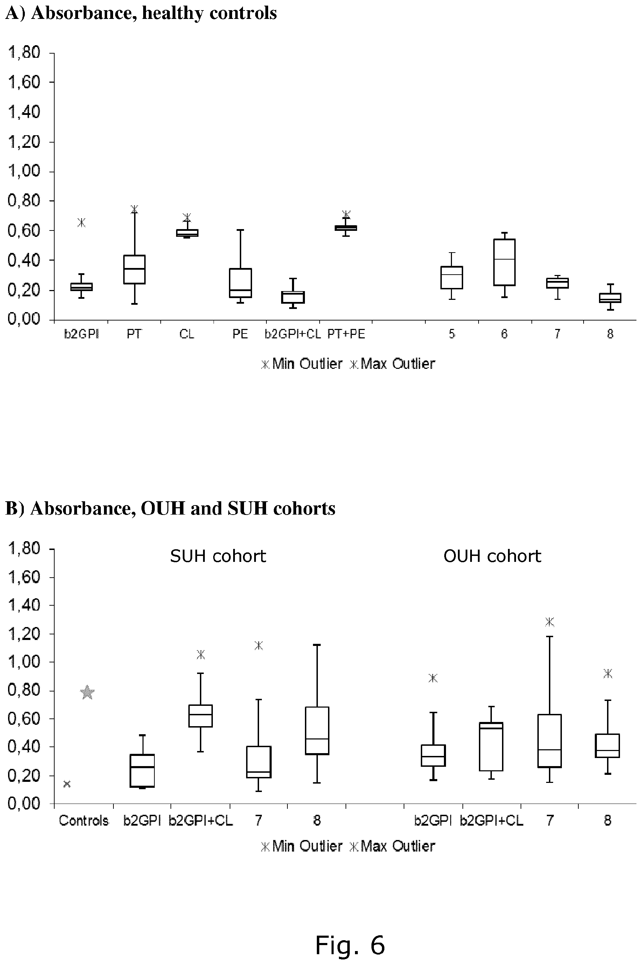

Summarized ELISA results for polyclonal control antibodies, healthy control sera and two patient cohorts are shown in Figure 6 ("5" and "6" coorespond to compound 6 and 7 respectively of Scheme VI). Negative and positive controls (HNP and HCL, Immunovision) are shown as a "x" and a star, respectively. As can be seen, the background signal of healthy controls was highest for cardiolipin 1, PT and mixture of phosphoethanolamine with PT. SUH samples showed higher aPLP titers than the OUH cohort, especially when antigen 8, Fig 5 and mixed antigen cardiolipin+beta2GPI were applied. Notably, aPLP positive samples indicated by compounds 7-8, Fig 5 dramatically varied from those indicated by separated antigens. Group analysis using ANOVA confirmed a statistically significant difference between the groups within SUH and OUH cohorts with a p value of 0.01. Finally, we analyzed medical records for the patients with respect to medication use. The data were divided into groups containing one or more medications and subjected to statistical analysis in Stata. Using two independent tests (Student's t-test and Fisher test), we confirmed no correlation between a particular medication or a group of medications and elevated aPLP titers.

### EXAMPLE 8 - Correlation between disease activity and aPLP titers for antigen 8 of figure 5

For statistical analysis, the following clinical parameters of each patient were considered: age (at onset and at sample date), gender, ethnicity, race, diagnosis, disease activity (at onset and at sample date), clinical manifestations, patient's complaints, and treatment history. Differences were analyzed for the antigens and controls reported in Table 1, using Student's t-test and ordinary least squares (OLS) analysis in Stata. Groups were compared for difference in means of antibody titers using ANOVA. A p-value of less than 0.05 was considered significant for each correlation.

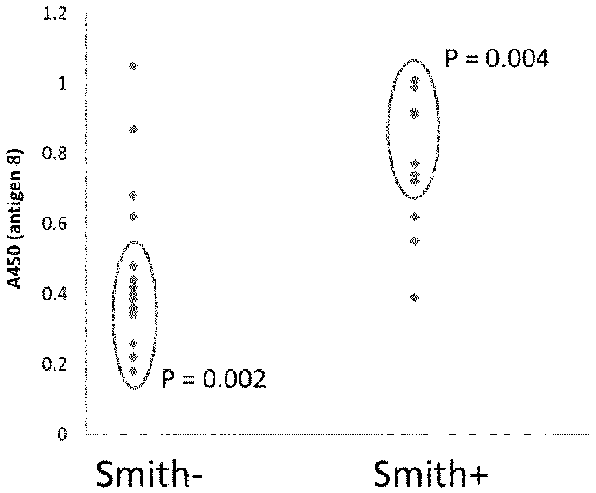

Students's t-test indicated a correlation between Smith positivity and elevated aPLP titers using exclusively antigen **8** (Figure 7).

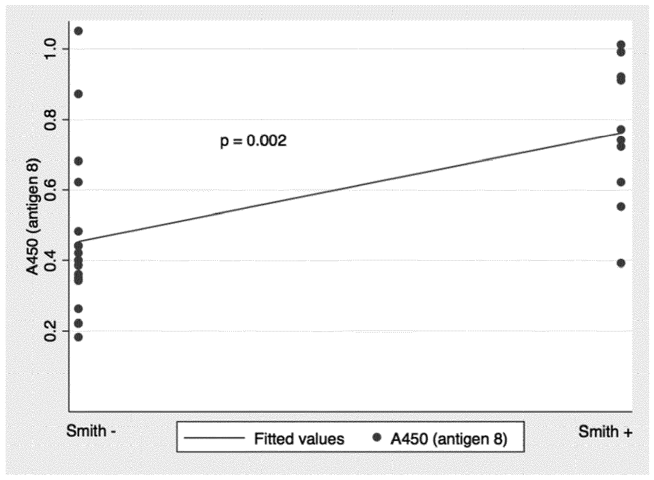

To further evaluate statistical difference between observed clinical parameters and aPLP titers, we performed ordinary least squares (OLS) analysis using Stata. The difference in aPLP titers for Smith negative and Smith positive groups was found to be statistically significant with p value of 0.002 (Figure 8).

Notably, no correlation with clinical parameters was observed for antigen 7 or other antigens and controls (p > 0.09). This implies uniqueness of the properties by antigen **8** and its potential in prognose of highly active autoimmune conditions which is indicated by Smith positivity.

### CONCLUSIONS

In this work, we introduce novel synthetic phospholipid-protein antigens, cardiolipin-beta2GPI and cardiolipin-PT, and prove their utility in diagnostics of autoantibodies. Novel antigens reported herein are prepared by convenient CuAAC click chemistry approach in high yields and purity (example 5). Tests of specificity against human monoclonal antibodies prove our hypothesis of improved specificity as a result of conjugating biologically complementary molecules in a regioselective fashion (example 6). Next, we show the utility of the prepared PLPs in enzyme-linked immunosorbent assay (ELISA) using patient samples and healthy controls (example 7). Finally, we prove correlation of the observed aPLPs with multiple clinical parameters and verify the causal effect of high disease activity on the elevated aPLP titers (example 8).

Our findings indicate higher prognostic potential of cross-linked PLPs than a simple mixture of its components or separate tests on each of them which are currently available on the market [37]. Moreover, the prepared antigens **7-8** (of figure 5)showed higher reproducibility and stability than currently available PL antigens and have advantage of straightforward synthetic route, high chemical homogeneity and decreased cross-reactivity on nonspecific human antibodies (Table 3).

### REFERENCES

- M. G. Paulick, A. R. Wise, M. B. Forstner, J. T. Groves, C. R. Bertozzi. J. Am. Chem. Soc. 2007, 129, 11543.
- WO 2012/134925 A1
- H. C. Hang, J. P. Wilson, G. Charron. Acc. Chem. Res. 2011, 44, 699.
- J. A. Precher, C. R. Bertozzy. Nat. Chem. Biol. 2005, 1, 13.
- M. Chassignol, Y. Aubert, V. Roig, U. Asseline. Nucleos. Nucleot. Nucl. Acids 2007, 26, 1669.
- A. M. Rouquette, C. Desgruelles. Lupus 2006, 15, 403, and references cited therein.
- A. Castro. H. Wang. WO 2007/061793 A2, PCT/US2006/044572.
- J. S. Yadav et al. Tetrahedron Lett. 1996, 37, 6603;
- K. Kasireddy et al. Bioorg. Chem. 2005, 33, 345.
- Molecules 2015, 20, 10253-10263

## Claims

1. A phospholipid-protein conjugate, wherein the conjugate is selected from compounds of formula BC-2 and BC-1: 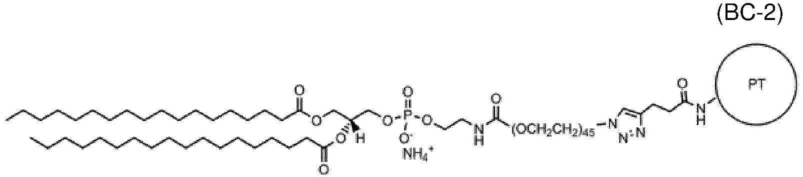 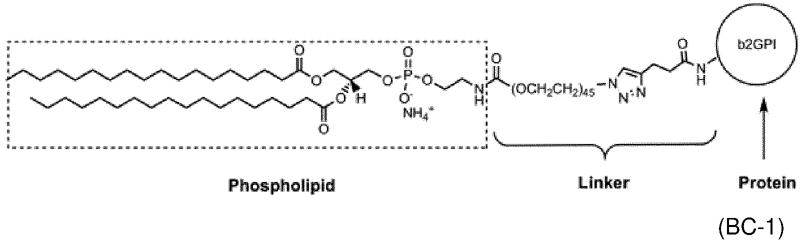 wherein b2GPI denotes β2-glycoprotein I, and PT denotes prothrombin.

2. The phospholipid-protein conjugate according to claim 1, wherein β2-glycoprotein I or prothrombin are bound to the linker via a terminal amino terminus or an amine-containing amino acid, such as lysine.

3. A method of detecting an autoantibody using the phospholipid conjugates according to any one of claims 1-2, wherein said method is an enzyme-linked immunosorbent assay (ELISA) method.

4. Use of the phospholipid conjugates according to any one of claims 1-2 for the identification of autoantibodies.

5. The method or use according to any one of claims 3 or 4, wherein the autoantibody is directed against human phospholipids or phospholipid-protein complexes.

6. The method or use according to any one of claims 3-5, wherein the autoantibody is an antibody involved in autoimmune diseases, such as antiphospholipid syndrome or systemic lupus erythematosus.

## Patentansprüche

1. Phospholipid-Protein Konjugat, wobei das Konjugat aus Verbindungen der Formel BC-2 und BC-1 ausgewählt ist: 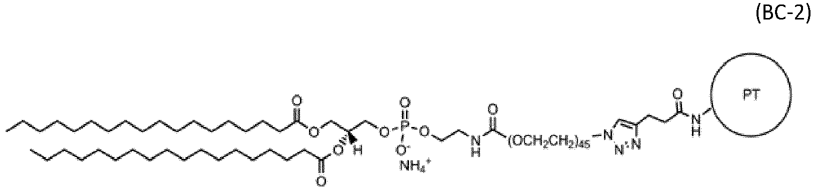 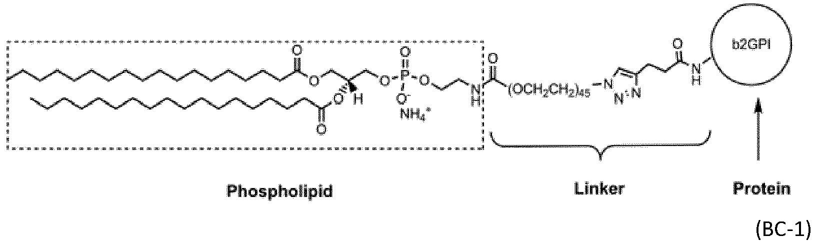 wobei b2GPI β2-Glykoprotein I bezeichnet und PT Prothrombin bezeichnet.

2. Phospholipid-Protein Konjugat nach Anspruch 1, wobei β2-Glykoprotein I oder Prothrombin über einen terminalen Aminoterminus oder eine aminhaltige Aminosäure, wie zum Beispiel Lysin, an den Linker gebunden sind.

3. Verfahren zum Nachweisen eines Autoantikörpers unter Verwendung der Phospholipid Konjugate nach einem der Ansprüche 1-2, wobei das Verfahren ein Enzyme Linked Immunosorbent Assay (ELISA)-Verfahren ist.

4. Verwendung der Phospholipid Konjugate nach einem der Ansprüche 1-2 zur Identifizierung von Autoantikörpern.

5. Verfahren oder Verwendung nach einem der Ansprüche 3 oder 4, wobei der Autoantikörper gegen humane Phospholipide oder Phospholipid-Protein-Komplexe gerichtet ist.

6. Verfahren oder Verwendung nach einem der Ansprüche 3-5, wobei der Autoantikörper ein Antikörper ist, der an Autoimmunerkrankungen beteiligt ist, wie zum Beispiel Antiphospholipid-Syndrom oder systemischer Lupus erythematodes.

## Revendications

1. Conjugué phospholipide-protéine, dans lequel le conjugué est choisi parmi les composés de formule BC-2 et BC-1 : 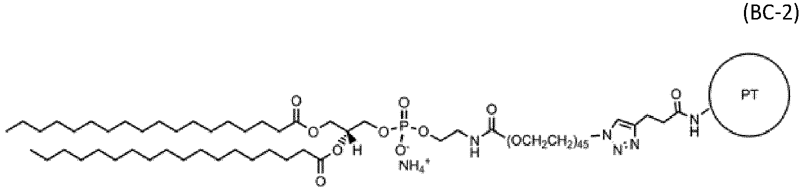 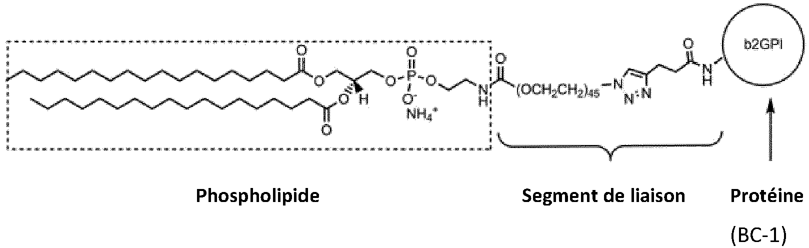 dans lequel b2GP1 signifie β2-glycoprotéine I, et PT signifie prothrombine.

2. Conjugué phospholipide-protéine selon la revendication 1, dans lequel la β2-glycoprotéine I ou la prothrombine sont liées au segment de liaison par une extrémité amino-terminale ou un acide aminé contenant un groupe amino, tel que la lysine.

3. Procédé de détection d'un auto-anticorps utilisant les conjugués de phospholipide selon l'une quelconque des revendications 1-2, dans lequel ledit procédé est un procédé de dosage par immunoadsorption à enzyme liée (ELISA).

4. Utilisation des conjugués de phospholipide selon l'une quelconque des revendications 1-2 pour l'identification d'auto-anticorps.

5. Procédé ou utilisation selon l'une quelconque des revendications 3 ou 4, dans lequel/laquelle l'auto-anticorps est dirigé contre des phospholipides ou complexes protéine-phospholipide humains.

6. Procédé ou utilisation selon l'une quelconque des revendications 3-5, dans lequel/laquelle l'auto-anticorps est un anticorps impliqué dans des maladies auto-immunes, telles que le syndrome des antiphospholipides ou le lupus sérythémateux disséminé.
